(19) Europäisches Patentamt

European Patent Office

Office européen des brevets

(11) Publication number: **0 388 262 B1**

(12) **EUROPEAN PATENT SPECIFICATION**

(45) Date of publication of patent specification: **28.09.94**  (51) Int. Cl.⁵: **C12N 1/18**, **A21D 8/04**

(21) Application number: **90400634.3**

(22) Date of filing: **09.03.90**

(54) **Novel Bakers' yeast.**

(30) Priority: **14.03.89 JP 59638/89**

(43) Date of publication of application:
**19.09.90 Bulletin 90/38**

(45) Publication of the grant of the patent:
**28.09.94 Bulletin 94/39**

(84) Designated Contracting States:
**AT BE CH DE DK ES FR GB GR IT LI LU NL SE**

(56) References cited:
**US-A- 4 547 374**

**PATENT ABSTRACTS OF JAPAN, vol. 13, no. 119 (C-579)[3467], 23rd March 1989;& JP-A-63 294 778**

(72) Inventor: **Takano, Hiroyuki**
**2-1318-702-201 Azuma**
**Tsukuba-shi, Ibaraki 305 (JP)**
Inventor: **Hino, Akihiro,**
**1410-804-303 Takezono**
**Tsukuba-shi, Ibaraki 305 (JP)**
Inventor: **Endo, Hisanori**
**B201 Coop. Wistaria,**
**3-7-5, Tokura**
**Mishima-shi, Shizuoka 411 (JP)**
Inventor: **Nakagawa, Nobuaki**
**865-8 Ohitocho-Ohito,**
**Tagata-gun**
**Shizuoka 410-23 (JP)**
Inventor: **Sato, Akio**
**1-17-14 Hikarigaoka**
**Mishima-shi, Shizuoka 411 (JP)**

(73) Proprietor: **NATIONAL FOOD RESEARCH IN-STITUTE, MINISTRY OF AGRICULTURE, FOR-ESTRY AND FISHERIES**
**2-1-2, Kannodai**
**Tsukuba-shi, Ibaraki (JP)**

Proprietor: **ASAHI KASEI KOGYO KABUSHIKI KAISHA**
**2-6, Dojimahama 1-chome**
**Kita-ku**
**Osaka-shi, Osaka 530 (JP)**

(74) Representative: **Bourgognon, Jean-Marie et al**
**Cabinet Flechner**
**22, Avenue de Friedland**
**F-75008 Paris (FR)**

**Description**

The present invention relates to a diploid hybrid strain characterized by at least having strong fermentative ability of "non-sugar bread dough" (hereinafter may be referred to as "non-sugar dough") and strong freeze-resistance and also relates to "frozen bread dough" (hereinafter may be referred to as "frozen dough") containing at least the hybrid strain and a dough composition. Furthermore, this invention relates to production of a variety of bread by using the frozen dough.

Recently, frozen dough for bread has been attractive so much in the baking industry because it has the following great advantages; i.e., (1) it may be useful in the supply of fresh-baked bread; and (2) it has vast merits for solving industrial laboring problems relating to time-shortening of a baking process (savings of labor) and termination of a night job. Frozen dough which is produced by kneading and fermenting materials for bread, keeping the material under freezing at around -20°C, until the time of baking after, if necessary, proofing. Common bakers' yeast is so likely damaged by fermentation prior to freezing that the use of yeast is limited to a case where dough enriched with a relatively large amount of materials such as sugar, fat, egg, milk products, etc. is not subjected to fermentation or subjected to fermentation for only a short time prior to freezing. When the dough containing common bakers' yeast which is through fermentation process for a short time before freezing is thawed and baked immediately after proofing, it cannot be sufficiently baked so that it causes some problems such that flavor and taste of bread may be deteriorated. A process wherein the frozen dough is thawed and subjected to proofing requires a long time for baking, so that the purpose of frozen dough method may be lost.

Thus, there has been a demand for a kind of bakers' yeast which has a great freeze-resistance, may be hardly damaged by storage under the frozen condition after fermentation, during prepared of frozen dough. Some reports have presented kinds of yeast having freeze-resistance, i.e., Saccharomyces rosei (Japanese Patent Kokoku No. 59-25584), Saccharomyces cerevisiae FTY (Japanese Patent Kokoku No. 59-48607), Saccharomyces cerevisiae IAM4274 (Japanese Patent Kokai No. 59-203442). Both Saccharomyces rosei and Saccharomyces cerevisiae FTY (FRI-413) do not have strong maltose-fermentative ability and therefore, they are not suitable for use in frozen dough with the sugar content in the range of 0 to 20 % by weight based on flour, i.e., from non-sugar dough to dough with a moderate sugar level. Saccharomyces cerevisiae IAM4274 has maltose-fermentative ability but does not show sufficient freeze-resistance in the dough with the sugar content in the range of 0 to 20 % by weight based on flour, i.e., from non-sugar dough, etc. to the dough with a moderate sugar level. Size of the yeast Saccharomyces rosei is smaller than that of common bakers' yeasts, so that it takes a long time for separation, washing and dehydration of yeasts. The other reports have presented bakers' yeast which is suitable for the dough for lean type bread and has maltose-fermentative ability and freeze-resistance, i.e. Saccharomyces cerevisiae KYF110 (Japanese Patent Kokai No. 62-208273), and a fusant strain Saccharomyces cerevisiae 3-2-6D (Japanese Patent Kokai No. 63-294778) which is obtained by the cell fusion technique and has strong maltose-fermentative ability and enhanced freeze-resistance. However, these are not satisfactory yet.

As heretofore mentioned, there has not been obtained bakers' yeast which has strong fermentative ability and great freeze-resistance of non-sugar dough up to the dough with a moderate sugar level (up to 20 % by weight based on flour), and which is appropriate for the non-sugar dough without sugar such as French bread and bread crumb or the dough for a white bread. Therefore, it has been extremely difficult to produce frozen dough for such kinds of bread.

The common bakers' yeast commercially available for non-sugar dough has a problem on storability because of the rapid decrease of its fermentative ability under storage in the form of products, compared with the deterioration rate of ordinary bakers' yeasts having high fermentative ability of dough of a higher sugar level and middle fermentative ability of non-sugar dough, which are widely used for the bread with the sugar content in the range of 5 % to 30 % by weight based on flour.

After intensive study to solve the above problems, the present inventors succeeded in a diploid hybrid strain which has both at least the same fermentative ability as the common bakers' yeasts for non-sugar dough, with respect to fermentation of dough having up to moderate sugar level (sugar content; 0 to 20 % by weight based on flour), and has strong freeze-resistance. The "diploid hybrid strain" is obtained by conjugation between a "haploid yeast strain" (hereinafter may be referred to as "haploid strain") obtained from germination of spores from a diploid yeast strain belonging to Saccharomyces cerevisiae having at least strong fermentative ability of non-sugar dough and a haploid yeast strain obtained from germination of spores from a diploid yeast strain belonging to Saccharomyces cerevisiae having so weak fermentative ability of non-sugar dough but strong freeze-resistance that it is hardly used for frozen dough of lean type bread. Furthermore, they found that the diploid hybrid strain was usable as bakers' yeast for non-sugar dough up to dough with a moderate sugar level, specifically for frozen dough. High quality bread can be obtained from frozen doughs which are made with the diploid hybrid strain and have non-sugar to a moderate sugar level, for example, non-sugar dough for such as French bread and bread crumb and dough for a white bread.

Additionally, the present diploid hybrid strain shows slower reduction in the fermentative ability during the storage than the common bakers' yeast for non-sugar dough. Thus, the diploid hybrid strain provides great advantages on storage.

According to the present invention, a diploid hybrid strain characterized by at least having strong fermentative ability of non-sugar dough and strong freeze-resistance, which is provided by conjugation between a haploid yeast strain obtained from germination of spores of a diploid yeast strain belonging to Saccharomyces cerevisiae having at least strong fermentative ability of non-sugar bread dough and a haploid yeast strain obtained from germination of spores from a diploid yeast strain belonging to Saccharomyces cerevisiae which has strong freeze-resistance but weak fermentative ability of non-sugar bread dough. The present invention further provides frozen bread dough containing at least the diploid hybrid strain and a dough composition.

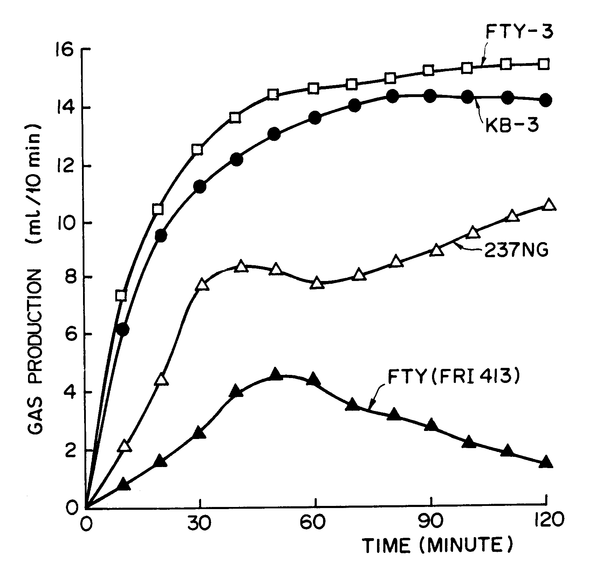

In accompanying drawing, it shows that $CO_2$ production every 10 minutes from non-sugar dough of Example 2. In the drawing, marks □, ●, △ and ▲ are for yeasts of the present diploid hybrid strain FTY-3 (FERM BP 2326), KB-3 (FERM BP 2742) or a mate for the FTY-3, 237NG and FTY (FERM BP 2743) or a mate for the FTY-3, respectively.

The diploid hybrid strain according to the present invention is obtained by conjugation between two parent strains, i.e. a haploid strain from germination of spores from a diploid yeast strain belonging to Saccharomyces cerevisiae which has at least strong fermentative ability of non-sugar dough and a haploid strain from germination of spores from a diploid yeast strain belonging to Saccharomyces cerevisiae which has strong freeze-resistance. One of them is Saccharomyces cerevisiae FTY-3 (FERM BP-2326) deposited at the Agency of Industrial Science and Technology, the Fermentation Research Institute, Japan.

One of the parent strains, a diploid yeast strain belonging to Saccharomyces cerevisiae having at least strong fermentative ability of non-sugar dough, may be any bakers' yeast for non-sugar dough commercially available in the market or any yeast strains which exhibit equivalent fermentative ability to the yeast above but has weak freeze-resistance. One of examples thereof is Saccharomyces cerevisiae KB-3 (FERM BP 2742; product name, "45 Red Yeast"; manufactured by Toyo Jozo Company, Ltd.).

Another parent diploid yeast strain belonging to Saccharomyces cerevisiae which has at least strong freeze-resistance, may be any yeast strains which have equivalent degree of freeze-resistance to that of the yeasts used in frozen rich bread dough and which exert weak fermentative ability of non-sugar dough but strong freeze-resistance. One of examples is Saccharomyces cerevisiae FTY (FRI-413) (FERM BP 2743).

Saccharomyces cerevisiae has the general properties mentioned below and the three diploid strains aforementioned above, namely, Saccharomyces cerevisiae FTY-3, Saccharomyces cerevisiae KB-3 and Saccharomyces cerevisiae FTY (FRI-413) have these characteristic properties.

| Fermentative ability of carbohydrates | |
|---|---|
| glucose | + |
| galactose | + |
| sucrose | + |
| maltose | + |
| lactose | - |
| Assimilation of carbon compounds | |
| glucose | + |
| galactose | + |
| sucrose | + |
| maltose | + |
| lactose | - |
| raffinose | + |
| soluble starch "Certified" (Lot. No. 0178-15, manufactured by Difco, Co. Ltd.) | + |
| Assimilation of nitrate ($KNO_3$) | - |
| Vitamin auxotrophy | |
| biotin | + |
| folic acid | - |
| nicotinic acid | - |
| thiamin | - |
| riboflavin | - |
| Ca-pantothenate | + |
| inositol | ± |
| pyridoxine | ± |
| p-aminobenzoic acid | - |

Separation of each haploid strain from the two parent strains is carried out by preculturing the parent strain each in a nutrient medium, inoculating the cells on a sporulation medium containing sodium acetate or potassium acetate, incubating the medium at 20 to 25°C for 2 to 7 days until spores are formed, suspending the cells containing spores in a lytic enzyme solution and keeping the solution to incubate at 30°C for 30 minutes to 1 hour. After the enzyme-treatment, spores are isolated from ascus using a micro manipulator. The spores isolated are transferred to the nutrient medium and cultured at 30°C until germination is made and haploid strain is obtained. Each haploid strain is judged by testing as to whether or not conjugation appears in the presence of a known mating type haploid strain.

Conjugation is conducted in such a manner as described in "Protein, Nucleic acid and Enzyme", Vol. 12, No. 12, pp. 1096-1099, Norio Gunke ed. (1967). Each haploid strain separated from the two parent strains is cultured in a nutrient medium at 30°C for 4 to 8 hours, separately, and then, the same amount of the each cultured medium is mixed and the mixture is incubated at 30°C, to generate zygotes. The zygotes formed are isolated with a micromanipulator and cultured. After several screenings of diploid hybrid strains having characteristics together of the parent strains. the present diploid hybrid strain Saccharomyces cerevisiae FTY-3 (FERM BP 2363) is obtained. The diploid hybrid strain was cultured with a fermenter and final product having water content of 64 - 73 % was obtained after dehydration.

In order to prepare the frozen dough, dough fomula such as flour, sugar, salt, fat, skim milk, egg, yeast food, yeast, water, etc., may be appropriately used. Sugar content in the dough is 0 - 20 % by weight based on flour. The diploid hybrid strain Saccharomyces cerevisiae FTY-3 as described above is produced in the compressed form of a 70 % water content, and then usually added to the dough in an amount of 1 to 10 % by weight based on flour.

The preparation of frozen dough, for instance, for a white bread, is conducted by a straight dough method wherein steps are as follows: mixing and kneading all of flour and other ingredients together with yeast, floor time, dividing, rounding, bench, intermediate proof, molding and freezing, sequentially. In a sponge dough method, steps are mixing and kneading a part of flour and other ingredients together with yeast, the first fermentation to prepare a sponge, and mixing and kneading the sponge with remaining ingredients, floor time, dividing, rounding, bench, molding and freezing, sequentially. In the case of straight dough method, the floor time is set at 0 to 240 minutes and frozen at -15 to -40°C, usually under the ambient pressure. The bread which is produced using the frozen dough of the present invention is quite

4

superior in apparency, specific volume, grain and flavor to the bread produced from frozen dough made with the common bakers' yeast.

The frozen dough of the present invention may be used for doughs of steamed pork buns, yeast doughnut and the like in addition to a variety of non-sugar dough having up to moderate sugar level for a white bread, French bread, bread crumb, buns or raisin bread.

The following examples explain the present invention but they are not intended to limit the present invention thereto.

Example 1 Conjugation

Production of the present diploid hybrid strain, Saccharomyces cerevisiae FTY-3, (hereinafter referred to as a FTY-3 strain).

Step 1. Presporulation

Each of Saccharomyces cerevisiae KB-3 (FERM BP 2742) having strong fermentative ability of non-sugar dough and Saccharomyces cerevisiae FTY (FRI-413) (FERM BP 2743) having strong freeze-resistance, was inoculated on a YPD agar medium plate and precultured at 30°C for 24 hours.

```
Composition of YPD agar medium (pH 5.5)
     yeast extract (Lot. 012701, manufactured by
     Difco, Co. Ltd.)                          5 g
     peptone (Lot 018802, manufactured by Difco,
     Co. Ltd.)                                 10 g
     glucose (special grade, manufactured by Wako
     Pure Chemical Industries, Ltd.)    40 g
```

$KH_2PO_4$ (Lot. CTJ3919, manufactured by Wako Pure Chemical Industries, Ltd.) 5 g

$MgSO_4 \cdot 7H_2O$ (Lot. CTP2140, manufactured by Wako Pure Chemical Industries, Ltd.) 2 g

```
     agar (Lot. 014001, manufactured by Difco,
     Co. Ltd.)                                 20 g
     distilled water                         1000 ml
```

Step 2. Sporulation

A loopful each of the two precultured strains was inoculated and cultured on Sherman's agar medium plate at 25°C for 6 days to induce sporulation.

```
         Composition of Sherman's agar medium (pH 7.2)
              ⎧ potassium acetate (special grade,
              ⎪ manufactured by Wako Pure Chemical
              ⎪ Industries, Ltd.)          1.0 g
              ⎪ yeast extract (Lot. 012701, manufactured
              ⎪ by Difco, Co. Ltd.)        0.1 g
              ⎨ glucose (special grade, manufactured
              ⎪ by Wako Pure Chemical Industries,
              ⎪ Ltd.)                      0.05 g
              ⎪ agar (Lot. 014001, manufactured by
              ⎪ Difco, Co. Ltd.)           2.0 g
              ⎩ distilled water            100 ml
```

Step 3. Separation of spores

A loopful each of the two sporulated strains was suspended in a solution (2 ml) of a lytic enzyme (product name "Zymolyase-20T"; $\beta$-1, 3-glacan lamunaripentaohydrase; 2 - 3U/ml, manufactured by KIRIN BREWERY, Co., Ltd.), respectively, and incubated at 30°C for 30 minutes to 1 hour. After the enzyme treatment, spores were isolated from ascus with a micromanipulator.

Step 4. Germination and production of haploid strains

Each spore isolated was placed on the YPD agar medium plate and cultured at 30°C until the spore was germinated and haploid strain was obtained. Mating type of strains were judged by testing as to whether or not conjugation appears in the presence of a known mating type haploid strains.

Step 5. Conjugation

Each haploid strain thus obtained, i.e. the haploid strain obtained from germination of the diploid yeast strain having strong fermentative ability of non-sugar dough and the haploid strain obtained from germination of spores of the diploid yeast strain having strong freeze-resistance, was separately cultured on the YPD medium, i.e., a medium having no agar in the YPD agar medium at 30°C for 4 to 8 hours. Each cultured medium was mixed together and incubated at 30°C until zygote was produced. The zygotes formed were isolated with a micromanipulator and cultured. Colony was formed on an agar medium plate containing maltose as a sugar source and the same agar medium was over laid to have it fermented. A colony which was able to produce the largest amount of $CO_2$ therearound was selected, and was cultured in a liquid medium containing molasses as a sugar source. Cells obtained were frozen at -20°C for 7 days. Cells after being thawed were allowed to ferment in a liquid fermenting medium containing maltose as a sugar source and a strain which was able to produce the largest amount of $CO_2$ was selected. The selected cell was mixed with non-sugar dough to prepare frozen dough. The dough was thawed and a strain which was able to produce a large amount of $CO_2$ was selected.

Thus, the present diploid hybrid strain, Saccharomyces cerevisiae FTY-3 strain was obtained.

Example 2 Fermentative ability of non-sugar dough

Each strain was allowed to ferment in dough defined below at 30°C for 2 hours and the $CO_2$ produced every 10 minutes for this period was measured as shown in the drawing attached.

6

EP 0 388 262 B1

```
Composition of non-sugar dough
⎧ flour (NISSHIN SEIFUN, Japan
⎪ trademark "Camelia" (bread making
⎪ protein flour)            20 g
⎨ salt                      0.3 g
⎪ yeast                     0.4 g
⎪ water (Shizuoka prefecture,
⎩ Ohito-cho)                13 ml
```

Table 1

|  | $CO_2$ production |
|---|---|
| ordinary bakers' yeast | 78 ml |
| yeast for non-sugar dough | 152 ml |
| FTY-3 strain | 161 ml |
| freeze-resistant yeast | 82 ml |
| FTY (FRI 413) | 32 ml |

Notes:
* Ordinary bakers' yeast; Saccharomyces cerevisiae 237NG (product name "45 Yeast", manufactured by Toyo Jozo Co., Ltd.).
* Yeast for non-sugar dough; Sacharomyces cerevisiae KB-3 (FERM BP 2742) (product name "45 Red Yeast", manufactured by Toyo Jozo Co., Ltd.).
* Freeze-resistant yeast; a product marketed in Japan.
* FTY (FRI 413); Saccharomyces cerevisiae FTY (FRI-413) (FERM BP 2743).

The same was applied to hereinafter.

Example 3 Fermentative ability of non-sugar dough and dough with a moderate sugar level

Each strain was fermented in two varieties of dough with the following composition, i.e. dough with a low sugar level (sugar content, 5 % based on flour) and dough with a moderate sugar level (sugar content, 20 % based on flour) at 30°C for 2 hours. $CO_2$ produced for this period was measured as shown in Table 2.

| Composition | | |
|---|---|---|
|  | Dough with a low sugar level | Dough with a moderate sugar level |
| flour (same above) | 20 g | 20 g |
| salt | 0.3 g | 0.3 g |
| sugar | 1 g | 4 g |
| yeast | 0.4 g | 0.4 g |
| water (same above) | 12.4 ml | 10 ml |

7

Table 2

| | CO$_2$ production in dough with a low sugar level | CO$_2$ production in dough with a moderate sugar level |
|---|---|---|
| ordinary bakers' yeast | 125 ml | 220 ml |
| yeast for non-sugar dough | 132 ml | 195 ml |
| FTY-3 strain | 136 ml | 215 ml |
| freeze-resistant yeast | 120 ml | 200 ml |

A strain FTY-3 showed weaker fermentative ability in dough with a high sugar level than in non-sugar dough and dough with a moderate sugar level.

Example 4 Freeze-resistance (non-sugar dough)

The non-sugar dough shown in Example 2 was fermentated at 30°C for 60 minutes and frozen at -20°C for 7 days. After being thawed at 30°C for 30 minutes, the resulting dough was fermentated at 38°C for 60 minutes and CO$_2$ produced was measured as shown in Table 3.

Table 3

| | CO$_2$ production before freezing | CO$_2$ production after thawing |
|---|---|---|
| ordinary bakers' yeast | 85 ml | 35 ml |
| yeast for non-sugar dough | 105 ml | 40 ml |
| FTY-3 strain | 108 ml | 88 ml |
| freeze-resistant yeast | 82 ml | 65 ml |

Example 5 Freeze-resistance (dough with a low sugar level)

The dough with a low sugar level shown in Example 3 was fermentated at 30°C for 60 minutes and frozen at -20°C for 7 days. After being thawed at 30°C for 30 minutes, the resulting dough was fermented at 30°C for 120 minutes. CO$_2$ produced in the latter fermentation was measured as shown in Table 4.

Table 4

| | CO$_2$ production before freezing | CO$_2$ production after thawing |
|---|---|---|
| ordinary bakers' yeast | 140 ml | 42 ml |
| yeast for non-sugar dough | 150 ml | 45 ml |
| FTY-3 strain | 154 ml | 118 ml |
| freeze-resistant yeast | 142 ml | 85 ml |

Example 6 Shelf life of yeast

CO$_2$ produced of the bakers' yeast for non-sugar dough was measured and that of a strain FTY-3, immediately after production thereof and after 4 days storage at 25°C. The results are shown in Table 3.

Table 5

|  | CO$_2$ production immediately after production | CO$_2$ production after strage |
|---|---|---|
| yeast for non-sugar dough FTY-3 strain | 150 ml<br>161 ml | 90 ml<br>142 ml |

Example 7 Sporulation and germination ratio

Each loopful strain as in Example 2 was thinly inoculated on the YM agar medium plate (10 ml per sterilized petri dish of 85 mm diameter) using a flame-sterilized loop and precultured at 30°C for 24 hours. The resulting each strain was again thinly inoculated on the Sharman's agar medium plate (10 ml per sterilized petri dish of 85 mm diameter) using a flame-sterilized loop and incubated at 25°C for 7 days.

The strain each was subsequently stained according to the Möller's method in "Classification and Identification of Yeast", pp. 17 - 18, Hiroshi Iizuka and Shoji Goto ed., published by Tokyo University Press, on March 21, 1969. The number of sporulating ascus every 100 cells was measured under a microscope.

Loopful cells each having sporulation ascus were suspended in a filtered solution (2 ml) of a lytic enzyme (3 mg/ml Zymolyase-20T in 0.05 M Tris-HCl buffer, pH 7.5), in a sterilized tube (18 mm diameter). The tubes were incubated at 30°C for 1 hour and centrifuged (3000 rpm) for 10 minutes. The obtained cells were washed twice with sterilized water and suspended in 2 ml of sterilized water.

Spores were isolated from the enzyme-treated cells with a micromanipulator and germinated at 30°C on the YNB agar medium plate, a synthetic medium for yeasts. One hundred spores were separated from asci having 4 spores each inside.

YM agar medium (pH 5.6)

yeast extract (Lot. 012701, manufactured by Difco, Co. Ltd.     0.3 g

glucose (special grade, manufactured by Wako Pure Chemical Industries, Ltd.)   1.0 g

maltose extract (Lot. 0186015, manufactured by Difco, Co. Ltd.)     0.3 g

peptone (Lot. 018802, manufactured by Difco, Co. Ltd.)     0.5 g

agar (Lot. 014001, manufactured by Difco, Co. Ltd.)     2.0 g

distilled water     100 ml

YNB agar medium (pH 5.6)
(Bacto-Yeast Nitrogen base in "Genetic experimental series, Vol. 3, Microbiological genetics research technique", pp. 186-188, Tatsuo Ishikawa ed., published by Kyoritsu Press, Japan, on March 10, 1982).

YEAST NITROGEN BASE (Lot. 760960, manufactured by Difco, Co. Ltd.)     0.67 g

glucose (special grade, manufactured by Wako Pure Chemical Industries, Ltd.)   2.0 g

agar (Lot. 014001, manufactured by Difco, Co. Ltd.)     2.0 g

distilled water     100 ml

The sporulation ratio and germination ratio of spores obtained above are shown in Table 6.

Table 6

| Strain | Sporulation ratio (%) | Germination ratio (%) |
|---|---|---|
| ordinary bakers' yeast | 2 | 4 |
| yeast for non-sugar dough | 20 | 10 |
| FTY-3 strain | 14 | < 1 |
| freeze-resistant yeast | 12 | 5 |
| FTY (FRI 413) | 2 | 8 |

The FTY-3 strain of the present invention as described above showed a characteristic that its germination ratio of spores was so small in the YNB agar medium plate, a synthetic medium for yeasts, that the strain could be identified from other strains.

EP 0 388 262 B1

Example 8 Serological characteristic of FTY-3 strain

Rabbit antisera generated against FTY-3 strain as antigen were immunologically examined in relation to each yeast strain as in Example 2.

The preparation of antigen and antibody and that of specific antibody absorbed with yeast strains (237 NG, KB-3, FTY-3 strain) were explained hereinafter. The agglutination was assayed as in the following.

Antigen: FTY-3 strain was suspended in physiological saline to a final concentration of about $10^{10}$ cells/ml and heated at 80°C for 30 minutes followed by centrifugation (1000 rpm) for 5 minutes to obtain 50 ml of the supernatant.

Antibody: Japanese white 5 rabbits aged 6 weeks were each injected 1 ml of the above antigen solution through ear vein using injectors. Additionally, 2 ml and 5 ml of the antigen solution were injected once and three times, respectively, with 4 days interval. On 8th day from the final immunization, each blood was taken out from carotid artery. Whole blood was centrifuged (3000 rpm) for 15 minutes to give about 20 ml of antiserum from each rabbit. Saturated ammonium sulfate solution (5 ml) was added to each antiserum (10 ml), and was kept at 5°C overnight and was centrifugated (5000 rpm) for 15 minutes. After each precipitated γ-globulin was dissolved and dialyzed against physiological saline, it was adjusted to a final protein concentration of 20 mg/ml and designated as an antibody solution (A).

Specific antibody preparations absorbed with 237NG, KB-3 or FTY-3 strain: The ordinary bakers' yeast (300 mg each, Saccharomyces cerevisiae 237NG) was added to antibody solutions (A) (2 ml each), and reaction was allowed to proceed at 5°C for 15 minutes. Supernatant obtained by centrifugation of the reaction solution was subjected to agglutination with the ordinary bakers' yeast, Saccharomyces cerevisiae 237NG. This process was repeated until no agglutination was observed. Subsequently, the resulting antibody solution was diluted with physiological saline to a final protein solution of 10 mg/ml (B).

Yeast (Saccharomyces cerevisiae KB-3) for non-sugar dough was used in place of the ordinary bakers' yeast to carry out the above procedure similarly. The resulting solution was adjusted to a protein solution of 10 mg/ml (C).

Furthermore, the antibody solution (C) was subjected to an absorption treatment with FTY-3 strain, and then, the protein concentration of the resulting antibody solution was adjusted to 10 mg/ml (D).

Procedures for agglutination test:

Each of the yeast cells was suspended in physiological saline to a concentration of about $10^9$ cells/ml. The suspension was divided to 40 μl each per well of 96-well microtiter plates. Each antibody solution (A) or any one of antibody solutions (B) through (D) was added to wells at a volume of 40 μl, separately, and shaken for 5 minutes. After these wells were kept to stand for 30 minutes, each well was examined in terms of the presence or absence of an agglutination mass. Tables 7, 8, 9 and 10 show the results of agglutination test using the antibody solution without absorption with yeast (A), the antibody solutions (B), (C) and (D), respectively. Normal serum shown in tables represents the serum from rabbits with no treatment.

(A)

Table 7

| Sample | Antibody solutions | | | | | |
|---|---|---|---|---|---|---|
| | 1 | 2 | 3 | 4 | 5 | Normal serum |
| ordinary bakers' yeast | + | + | + | + | + | - |
| yeast for non-sugar dough | + | + | + | + | + | - |
| FTY-3 strain | + | + | + | + | + | - |
| freeze-resistant yeast | + | + | + | + | + | - |
| FTY (FRI 413) | + | + | + | + | + | - |

(B)

Table 8

| Sample | Antibody solutions | | | | | |
|---|---|---|---|---|---|---|
| | 1 | 2 | 3 | 4 | 5 | Normal serum |
| ordinary bakers' yeast | - | - | - | - | - | - |
| yeast for non-sugar dough | - | + | + | - | - | - |
| FTY-3 strain | - | + | + | + | - | - |
| freeze-resistant yeast | - | - | - | - | - | - |
| FTY (FRI 413) | - | + | + | + | - | - |

(C)

Table 9

| Sample | Antibody solutions | | | | | |
|---|---|---|---|---|---|---|
| | 1 | 2 | 3 | 4 | 5 | Normal serum |
| ordinary bakers' yeast | - | - | - | - | - | - |
| yeast for non-sugar dough | - | - | - | - | - | - |
| FTY-3 strain | - | + | + | + | + | - |
| freeze-resistant yeast | - | - | - | - | - | - |
| FTY (FRI 413) | - | + | + | + | - | - |

(D)

Table 10

| Sample | Antibody solutions | | | | | |
|---|---|---|---|---|---|---|
| | 1 | 2 | 3 | 4 | 5 | Normal serum |
| ordinary bakers' yeast | - | - | - | - | - | - |
| yeast for non-sugar dough | - | - | - | - | - | - |
| FTY-3 strain | - | - | - | + | + | - |
| freeze-resistant yeast | - | - | - | - | - | - |
| FTY (FRI 413) | - | - | - | - | - | - |

Example 9 Baking test

1 French bread

```
Composition
⎧ flour (same above)              100 g
⎪ yeast                          4 g
⎪ yeast foods (Toyo Jozo, trade
⎪              name "Amila")      0.1 g
⎨ malt extract (SANKYO FOODS,
⎪              Japan, trade name
⎪              "Sankyo Malt Ekisu
⎪              B₂")               0.3 g
⎪ salt                           2 g
⎩ water (same above)             63 ml
```

```
Procedure (Straight dough method)
⎧ fermentation time              60 min.
⎪ dividing                       100 g
⎪ bench time                     20 min.
⎪ freezing after molding at -20°C
⎨ thawing                  30°C, 60 min.
⎪ proofing                 20°C, 60 min.
⎩ baking                  230°C, 15 min.
```

Table 11

| | Bread volume | | |
|---|---|---|---|
| | non-freezing | frozen for 7 days | frozen for 14 days |
| yeast for non-sugar dough | 350 ml | 270 ml | 250 ml |
| FTY-3 strain | 360 ml | 310 ml | 300 ml |
| freeze-resistant yeast | 310 ml | 280 ml | 275 ml |

13

2 A white bread (sugar content: 5 % based on flour)

```
Composition
┌ flour (same above)              100 g
│ yeast                           4 g
│ yeast foods (same above)        0.1 g
│ fat                             5 g
│ sugar                           5 g
│ salt                            2 g
└ water (same above)              65 ml


Procedure (straight dough method)
┌ fermentation time         40 min.
│ dividing                  50 g
│ freezing after molding at -20°C
│ thawing                   30°C, 90 min.
│ proofing                  38°C, 40 min.
└ baking                    200°C, 35 min.
```

Table 12

| | Bread volume | | |
|---|---|---|---|
| | non-freezing | frozen for 7 days | frozen for 14 days |
| yeast for non-sugar dough | 285 ml | 180 ml | 145 ml |
| FTY-3 strain | 290 ml | 265 ml | 230 ml |
| freeze-resistant yeast | 280 ml | 230 ml | 195 ml |

3 Butter roll (sugar content: 10 % based on flour)

```
Composition
 ⎧  flour (same above)          100 g
 ⎪  yeast                       4 g
 ⎪  yeast foods (same above)    0.1 g
 ⎪  fat                         10 g
 ⎨  sugar                       10 g
 ⎪  whole egg                   10 g
 ⎪  salt                        1.5 g
 ⎩  water (same above)          48 ml


Procedure (straight dough method)
 ⎧  fermentation time           45 min.
 ⎪  dividing                    50 g
 ⎪  first bench time            10 min.
 ⎨  second bench time           10 min.
 ⎪  molding
 ⎪  freezing after molding at -20°C
```

```
 ⎧  thawing         30°C, 30 min.
 ⎪  proofing        38°C, 80 % humidity,
 ⎨                  40 min.
 ⎩  baking          200°C, 10 min.
```

Table 13

| | Bread volume | | |
|---|---|---|---|
| | non-freezing | frozen for 7 days | frozen for 14 days |
| yeast for non-sugar dough | 255 ml | 185 ml | 130 ml |
| FTY-3 strain | 270 ml | 255 ml | 240 ml |
| freeze-resistant yeast | 250 ml | 220 ml | 170 ml |

15

4 Buns (sugar content: 15 % based on flour)

```
                    Composition
          ┌ flour (same above)        100 g,
          │ skim milk                 3 g
          │ yeast                     4 g
          │ whole egg                 8 g
          │ yeast food (same above)   0.1 g
          │ fat                       8 g
          │ sugar                     15 g
          │ salt                      1.3 g
          └ water (same above)        50 ml


          Procedure (straight dough method)
          ┌ fermentation time         40 min.
          │ dividing                  50 g
          │ bench time                15 min.
          │ freezing after molding at -20°C
          │ thawing                   30°C, 30 min.
          │ proofing                  38°C, 50 min.
          └ baking                    200°C, 10 min.
```

Table 14

|  | Bread volume | | |
|---|---|---|---|
|  | non-freezing | frozen for 7 days | frozen for 14 days |
| yeast for non-sugar dough<br>FTY-3 strain<br>freeze-resistant yeast | 280 ml<br>282 ml<br>270 ml | 250 ml<br>278 ml<br>265 ml | 240 ml<br>273 ml<br>260 ml |

5 Buns (sugar content: 20 % based on flour)

```
            Composition
          ⎧ flour (same above)        100 g,
          ⎪ skim milk                   3 g
          ⎪ yeast                       5 g
          ⎨ whole egg                   8 g
          ⎪ yeast food (same above)   0.1 g
          ⎪ fat                         8 g
          ⎩ sugar                      20 g


          ⎧ salt                      1.3 g
          ⎩ water (same above)       48 ml


            Procedure (straight dough method)
          ⎧ fermentation time         60 min.
          ⎪ dividing                  50 g
          ⎪ bench time                15 min.
          ⎨ freezing after molding at -20°C
          ⎪ thawing                   30°C, 30 min.
          ⎪ proofing                  38°C, 50 min.
          ⎩ baking                    200°C, 10 min.
```

Table 15

|  | Bread volume | | |
|---|---|---|---|
|  | non-freezing | frozen for 7 days | frozen for 14 days |
| yeast for non-sugar dough | 300 ml | 265 ml | 242 ml |
| FTY-3 strain | 293 ml | 292 ml | 290 ml |
| freeze-resistant yeast | 276 ml | 270 ml | 265 ml |

According to the present invention, high-quality frozen bread dough having non-sugar for French bread and bread crumb and white bread having up to a moderate sugar level (sugar content: 0 to 20 % based on flour) may be provided.

Furthermore, non-sugar dough containing the diploid hybrid strain according to the present invention has longer storage life than the same dough containing the conventional yeasts. Accordingly, the present yeast or dough containing the same is convenient when stored or transported.

**Claims**
**Claims for the following Contracting States : AT, BE, CH, DE, DK, FR, GB, GR, IT, LI, LU, NL, SE**

1. A diploid hybrid strain characterized by at least having strong fermentative ability of non-sugar bread dough and strong freeze-resistance, which is obtained by conjugation between a haploid yeast strain obtained from germination of spores from a diploid yeast strain belonging to <u>Saccharomyces cerevisiae</u> which has at least strong fermentative ability of non-sugar bread dough and a haploid yeast strain obtained from germination of spores from a diploid yeast strain belonging to <u>Saccharomyces cerevisiae</u> which has weak fermentative ability of non-sugar bread dough but strong freeze-resistance.

2. The diploid hybrid strain according to claim 1, wherein the diploid hybrid strain is <u>Saccharomyces cerevisiae</u> FTY-3 (FERM BP-2326).

3. Frozen bread dough containing at least a bread dough composition and the diploid hybrid strain according to claim 1.

4. Frozen bread dough according to claim 3, wherein sugar added to the bread dough composition is 0 to 20 % based on flour.

**Claims for the following Contracting State : ES**

1. Process for obtaining a diploid hybrid strain having strong fermentative ability of non-sugar bread dough and strong freeze-resistance, comprising carrying out a conjugation between a haploid yeast strain obtained from germination of spores from a diploid yeast strain belonging to <u>Saccharomyces cerevisiae</u> which has at least strong fermentative ability of non-sugar bread dough and a haploid yeast strain obtained from germination of spores from a diploid yeast strain belonging to <u>Saccharomyces cerevisiae</u> which has weak fermentative ability of non-sugar bread dough but strong freeze-resistance.

2. The process according to claim 1, wherein the diploid hybrid strain is <u>Saccharomyces cerevisiae</u> FTY-3 (FERM BP-2326).

3. Frozen bread dough containing at least a bread dough composition and a diploid hybrid strain having strong fermentative ability of non-sugar bread dough and strong freeze-resistance, which is obtained by conjugaison between a haploid yeast strain obtained from germination of spores from a diploid yeast strain belonging to <u>Saccharomyces cerevisiae</u> which has at least strong fermentative ability of non-sugar bread dough and a haploid yeast strain obtained from germination of spores from a diploid yeast strain belonging to <u>Saccharomyces cerevisiae</u> which has weak fermentative ability of non-sugar bread dough but strong freeze-resistance.

4. Frozen bread dough according to claim 3, wherein sugar added to the bread dough composition is 0 to 20 % by weight based on flour.

5. Process for preparing frozen bread dough comprising mixing a bread dough composition and a diploid hybrid strain having strong fermentative ability of non-sugar bread dough and strong freeze-resistance, which is obtained by conjugaison between a haploid yeast strain obtained from germination of spores from a diploid yeast strain belonging to <u>Saccharomyces cerevisiae</u> which has at least strong fermentative ability of non-sugar bread dough and a haploid yeast strain obtained from germination of spores from a diploid yeast strain belonging to <u>Saccharomyces cerevisiae</u> which has weak fermentative ability of non-sugar bread dough but strong freeze-resistance.

6. Process according to claim 5, comprising adding sugar to the bread dough composition in an amount of 0 to 20% by weight based on the flour of the composition.

**Patentansprüche**
**Patentansprüche für folgende Vertragsstaaten : AT, BE, CH, DE, DK, FR, GB, GR, IT, LI, LU, NL, SE**

1. Diploider Hybridstamm, **gekennzeichnet** durch zumindest starkes Fermentationsvermögen für nicht-gezuckerten Brotteig und starke. Kältebeständigkeit, wobei der Hybridstamm durch Konjugieren zwischen einem haploiden Hefestamms, der durch Sporenkeimen eines diploiden Hefestamms erhalten worden ist, der zu *Saccharomyces cerevisiae* gehört und mindestens starkes Fermentationsvermögen für nicht-gezuckerten Brotteig besitzt, und einem haploiden Hefestamm erhalten worden ist, der durch Sporenkeimen eines diploiden Hefestamms erhalten worden ist, der zu *Saccharomyces cerevisiae* gehört und schwaches Fermentationsvermögen für nicht-gezuckerten Brotteig, jedoch starke Kältebeständigkeit besitzt.

2. Diploider Hybridstamm nach Anspruch 1, wobei der diploide Hybridstamm *Saccharomyces cerevisiae* FTY-3 (FERM BP 2326) ist.

3. Gefrorener Brotteig, enthaltend mindestens eine Brotteig-Zu sammensetzung und den diploiden Hybridstamm gemäß Anspruch 1.

4. Gefrorener Brotteig nach Anspruch 3, bei dem Zucker, der zur Brotteig-Zusammensetzung zugegeben worden ist, 0 bis 20 Gew.-% auf Basis des Mehls ausmacht.

**Patentansprüche für folgenden Vertragsstaat : ES**

1. Verfahren zur Gewinnung eines diploiden Hybridstammes mit starkem Fermentationsvermögen für nicht-gezuckerten Brotteig und starker Kältebeständigkeit, bei dem man eine Konjugation zwischen einem haploiden Hefestamm, der durch Sporenkeimen eines diploiden Hefestamms erhalten worden ist, der zu *Saccharomyces cerevisiae* gehört und mindestens starkes Fermentationsvermögen für nicht-gezuckerten Brotteig besitzt, und einem haploiden Hefestamm durchführt, der durch Sporenkeimen eines diploiden Hefestamms erhalten worden ist, der zu *Saccharomyces cerevisiae* gehört und schwaches Fermentationsvermögen für nicht-gezuckerten Brotteig, jedoch starke Kältebeständigkeit besitzt.

2. Verfahren nach Anspruch 1, bei dem der diploide Hybridstamm *Saccharomyces cerevisiae* FTY-3 (FERM BP-2326) ist.

3. Gefrorener Brotteig mit einem Gehalt an mindestens einer Brotteig-Zusammensetzung und einem diploiden Hybridstamm mit starkem Fermentationsvermögen für nicht-gezuckerten Brotteig und starker Kältebeständigkeit, wobei der Hybridstamm durch Konjugieren zwischen einem haploiden Hefestamm, der durch Sporenkeimen eines diploiden Hefestamms erhalten worden ist, der zu *Saccharomyces cerevisiae* gehört und mindestens starkes Fermentationsvermögen für nicht-gezuckerten Brotteig besitzt, und einem haploidem Hefestamm erhalten worden ist, der durch Sporenkeimen eines diploiden Hefestamms erhalten worden ist, der zu *Saccharomyces cerevisiae* gehört und schwaches Fermentationsvermögen für nicht-gezuckerten Brotteig, jedoch starke Kältebeständigkeit besitzt.

4. Gefrorener Brotteig nach Anspruch 3, bei dem Zucker, der zur Brotteig-Zusammensetzung zugegeben worden ist, 0 bis 20 Gew.-% auf Basis des Mehls ausmacht.

5. Verfahren zur Herstellung von gefrorenem Brotteig, bei dem man eine Brotteig-Zusammensetzung und einen diploiden Hybridstamm mit starkem Fermentationsvermögen für nicht-gezuckerten Brotteig und starker Kältebeständigkeit mischt, wobei der Hybridstamm durch Konjugation zwischen einem haploiden Hefestamm, der durch Sporenkeimen eines diploiden Hefestamms, der zu *Saccharomyces cerevisiae* gehört und mindestens starkes Fermentationsvermögen für nicht-gezuckerten Brotteig besitzt, und einem haploiden Hefestamm erhalten worden ist, der durch Sporenkeimen eines diploiden Hefestamms erhalten worden ist, der zu *Saccharomyces cerevisiae* gehört und schwaches Fermentationsvermögen für nicht-gezuckerten Brotteig, jedoch starke Kältebeständigkeit besitzt.

6. Verfahren nach Anspruch 5, bei dem man Zucker zur Brotteig-Zusammensetzung in einer Menge von 0 bis 20 Gew.-% auf Basis des Mehls der Zusammensetzung zugibt.

**Revendications**
**Revendications pour les Etats contractants suivants : AT, BE, CH, DE, DK, FR, GB, GR, IT, LI, LU, NL, SE**

1. Souche diploïde hybride, caractérisée en ce qu'elle a au moins une grande aptitude à la fermentation de la pâte à pain non sucrée et une grande résistance à la congélation, qui est obtenue en conjuguant une source haploïde de levure, obtenue par germination de spores d'une souche diploïde de levure appartenant à Saccharomyces cerevisiae, qui a au moins une grande aptitude à la fermentation de la pâte à pain non sucrée, et une source haploïde de levure, obtenue par germination de spores d'une souche diploïde de levure appartenant à Saccharomyces cerevisiae, qui a une faible aptitude à la fermentation d'une pâte à pain non sucrée, mais une grande résistance à la congélation.

2. La souche diploïde suivant la revendication 1, dans laquelle la souche diploïde hybride est Saccharomyces cerevisiae FTY-3 (FERM BP-2326).

3. Pâte à pain congelée contenant au moins une composition de pâte à pain et la souche diploïde hybride suivant la revendication 1.

4. Pâte à pain congelée suivant la revendication 3, dans laquelle le sucre ajouté à la composition de pâte à pain représente de 0 à 20 % de la farine.

**Revendications pour l'Etat contractant suivant : ES**

1. Procédé d'obtention d'une souche diploïde hybride ayant une grande aptitude à la fermentation d'une pâte à pain non sucrée et une grande résistance à la congélation, qui consiste à conjuguer une source haploïde de levure, obtenue par germination de spores d'une souche diploïde de levure appartenant à Saccharomyces cerevisiae, qui a au moins une grande aptitude à la fermentation de la pâte à pain non sucrée, et une source haploïde de levure, obtenue par germination de spores d'une souche diploïde de levure appartenant à Saccharomyces cerevisiae, qui a une faible aptitude à la fermentation d'une pâte à pain non sucrée, mais une grande résistance à la congélation.

2. Procédé suivant la revendication 1, dans lequel la souche diploïde hybride est Saccharomyces cerevisiae FTY-3 (FERM BP-2326).

3. Pâte à pain congelée contenant au moins une composition de pâte à pain et une souche diploïde hybride ayant une grande aptitude à la fermentation d'une pâte à pain non sucrée et une grande résistance à la congélation, qui est obtenue en conjugant une source haploïde de levure, obtenue par germination de spores d'une souche diploïde de levure appartenant à Saccharomyces cerevisiae qui a au moins une grande aptitude à la fermentation de la pâte à pain non sucrée et une source haploïde de levure, obtenue par germination de spores d'une souche diploïde de levure appartenant à Saccharomyces cerevisiae, qui a une faible aptitude à la fermentation d'une pâte à pain non sucrée, mais une grande résistance à la congélation.

4. Pâte à pain congelée suivant la revendication 3, dans laquelle le sucre ajouté à la composition de pâte à pain représente de 0 à 20 % du poids de la farine.

5. Procédé de préparation d'une pâte à pain congelée qui consiste à mélanger une composition de pâte à pain et une souche diploïde hybride ayant une grande aptitude à la fermentation d'une pâte à pain non sucrée et une grande résistance à la congélation, qui est obtenue en conjugant une source haploïde de levure, obtenue par germination de spores d'une souche diploïde de levure appartenant à Saccharomyces cerevisiae qui a au moins une grande aptitude à la fermentation de la pâte à pain non sucrée, et une source haploïde de levure, obtenue par germination de spores d'une souche diploïde de levure appartenant à Saccharomyces cerevisiae, qui a une faible aptitude à la fermentation d'une pâte à pain non sucrée, mais une grande résistance à la congélation.

6. Procédé suivant la revendication 5, qui consiste à ajouter du sucre à la composition de pâte à pain en une quantité représentant de 0 à 20 % du poids de la farine de la composition.

20